(19) Europäisches Patentamt
European Patent Office
Office européen des brevets

(11) EP 1 699 751 B1

(12) EUROPÄISCHE PATENTSCHRIFT

(45) Veröffentlichungstag und Bekanntmachung des Hinweises auf die Patenterteilung:
**02.03.2011 Patentblatt 2011/09**

(21) Anmeldenummer: **04804184.2**

(22) Anmeldetag: **22.12.2004**

(51) Int Cl.:
***C07C 209/16*** (2006.01) ***C07C 29/76*** (2006.01)
***C12P 7/06*** (2006.01)

(86) Internationale Anmeldenummer:
**PCT/EP2004/014588**

(87) Internationale Veröffentlichungsnummer:
**WO 2005/063681 (14.07.2005 Gazette 2005/28)**

(54) **VERFAHREN ZUR HERSTELLUNG EINES ETHYLAMINS**

METHOD FOR PRODUCING A ETHYLAMINE

PROCEDE DE FABRICATION D'UNE ETHYLAMINE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **23.12.2003 DE 10361503**

(43) Veröffentlichungstag der Anmeldung:
**13.09.2006 Patentblatt 2006/37**

(73) Patentinhaber: **BASF SE**
**67056 Ludwigshafen (DE)**

(72) Erfinder:
- **MEIER, Anton**
**67134 Birkenheide (DE)**
- **MELDER, Johann-Peter**
**67459 Böhl-Iggelheim (DE)**
- **GERLACH, Till**
**67071 Ludwigshafen (DE)**
- **HAESE, Frank**
**24855 Bollingstedt (DE)**

(56) Entgegenhaltungen:
**EP-A- 0 101 254** **EP-A- 0 211 552**
**CA-A1- 1 242 221** **US-A- 4 690 903**

- **PATENT ABSTRACTS OF JAPAN Bd. 010, Nr. 245 (C-368), 22. August 1986 (1986-08-22) & JP 61 074568 A (K F ENG KK), 16. April 1986 (1986-04-16)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 699 751 B1

## Beschreibung

Die vorliegende Erfindung betrifft ein Verfahren zur Herstellung eines Ethylamins durch Umsetzung von Ethanol mit Ammoniak, einem primären Amin oder einem sekundären Amin in Gegenwart von Wasserstoff und eines Heterogenkatalysators.

Die Herstellung von Ethylaminen (Mono-, Di- und Triethylamin) erfolgt technisch durch Aminierung von Ethanol mit Ammoniak, primären oder sekundären Aminen in Gegenwart von Wasserstoff.

Verfahren zur Herstellung von Aminen aus Alkoholen sind dem Fachmann aus der Literatur, z.B. Ullmann's Encyclopedia of Industrial Chemistry, 6th Edition, 2000 electronic release, ‚aliphatic Amines: Production from alcohols', bekannt.

Das eingesetzte Ethanol kann synthetisch hergestellt werden, etwa durch Hydratisierung von Ethylen. Als Alternative zu synthetischem Ethanol bietet sich biochemisch, insbesondere fermentativ hergestelltes, sogenanntes Bio-Ethanol, an. Dieses wird aus regenerativen Quellen hergestellt und ist damit aus ökologischen Gründen vorteilhaft. Zudem weist Bio-Ethanol teilweise einen günstigeren Preis auf als synthetisches Ethanol.

Beim Einsatz von Bio-Ethanol an einem typischen Aminierungs-Katalysator (z.B. einem Cu/Co/Ni-Katalysator geträgert auf gamma-$Al_2O_3$) wurde jedoch eine deutlich schnellere Katalysatordesaktivierung beobachtet als dies bei Einsatz von synthetischem Ethanol bekannt war.

Aufgrund der schnelleren Desaktivierung muss die Synthese häufiger unterbrochen werden, um den Katalysator zu wechseln. Dies führt zu Produktionsausfall, erhöhten Kosten für Katalysator und Katalysatorwechsel und einem erhöhten Personalaufwand verbunden mit erhöhtem Unfallrisiko.

Wird Bio-Ethanol in Aminierungsverfahren eingesetzt, belegt sich, wie erfindungsgemäß erkannt wurde, die katalytisch aktive Metalloberfläche des jeweiligen Heterogenkatalysators mit der Zeit mehr und mehr mit dem/den durch den Bio-Alkohol eingetragenen Schwefel bzw. Schwefelverbindungen. Dies führt zu einer beschleunigten Katalysatordesaktivierung und damit zu einer deutlichen Beeinträchtigung der Wirtschaftlichkeit des jeweiligen Prozesses.

Es bestand also das Problem, den Schwefel und/oder die schwefelhaltigen Verbindungen, insbesondere die störenden schwefelhaltigen Verbindungen, in Bio-Ethanol durch eine vorgelagerte Entschwefelungsstufe abzureichern oder praktisch ganz zu entfernen.

Die Reinigung bzw. Isolierung von biochemisch hergestellten Verbindungen, wie Bio-Ethanol, erfolgt häufig destillativ in aufwendigen, mehrstufigen Verfahren.

WO-A-2003 020850, US-A1-2003 070966, US.A1-2003 113598 und US-B1-6,531,052 betreffen die Entfernung von Schwefel aus flüssiges Kohlenwasserstoffen (Benzinen).

EP-A-0101254 beschreibt eine Prozess zur Abtrennung von Ethanol aus verdünnten wässrigen Lösungen. Chemical Abstracts Nr. 102: 222463 (M.Kh. Annagiev et al., Doklady - Akademiya Nauk Azerbaidzhanskoi SSR, 1984, 40 (12), 53-6) beschreibt die Abreicherung von S-Verbindungen aus technischem Ethanol (nicht Bio-Ethanol) von 25-30 auf 8-17 mg/l durch In-Kontakt-Bringen des Ethanols bei Raumtemperatur mit Zeolithen von Clinoptilolit- und Mordenit-Typ, wobei diese Zeolithe zuvor bei 380 °C, 6 h konditioniert wurden und in einigen Fällen mit Metallsalzen, insbesondere $Fe_2O_3$, behandelt wurden. Bei den abgereicherten S-Verbindungen handelt es sich um $H_2S$ und Alkylthiole (R-SH).

Der vorliegenden Erfindung lag die Aufgabe zugrunde, ein verbessertes wirtschaftliches Verfahren zur katalytischen Herstellung eines Ethylamins durch Umsetzung von Ethanol mit Ammoniak, einem primären Amin oder einem sekundären Amin aufzufinden, durch das in hoher Ausbeute, Raum-Zeit-Ausbeute und Selektivität entsprechende Ethylamine, insbesondere Mono-, Di- und Triethylamin, erhalten werden. Insbesondere sollte das Verfahren verlängerte Katalysatorstandzeiten bei der Synthese der Ethylamine ermöglichen.

(Raum-Zeit-Ausbeuten werden angegeben in ‚Produktmenge / (Katalysatorvolumen • Zeit)' (kg/($l_{Kat.}$ • h)) und/oder ‚Produktmenge / (Reaktorvolumen • Zeit)' (kg/($l_{Reaktor}$ • h)).

Demgemäß wurde ein Verfahren zur Herstellung eines Ethylamins (oder eines Gemisches von Ethylaminen) durch Umsetzung von Ethanol mit Ammoniak, einem primären Amin oder einem sekundären Amin in Gegenwart von Wasserstoff und eines Heterogenkatalysators gefunden, welches dadurch gekennzeichnet ist, dass man ein biochemisch hergestelltes Ethanol (= Bio-Ethanol) einsetzt, in welchem zuvor durch In-Kontakt-Bringen mit einem Adsorber Schwefel und/oder schwefelhaltige Verbindungen abgereichert wurden.

Bei den schwefelhaltigen Verbindungen handelt es sich um anorganische oder organische Verbindungen, insbesondere um symmetrische oder unsymmetrische $C_{2-10}$-Dialkylsulfide, besonders $C_{2-6}$-Dialkylsulfide, wie Diethylsulfide Di-n-propylsulfid, Di-isopropylsulfid, ganz besonders Dimethylsulfid, $C_{2-10}$-Dialkylsulfoxide, wie Dimethylsulfoxid,

Diethylsulfoxid, Dipropylsulfoxid, 3-Methylthio-1-propanol und/oder S-haltigen Aminosäuren, wie Methionin und S-Methyl-methionin.

Als Adsorber werden ein Kieselgel, ein aktiviertes Aluminiumoxid, ein Zeolith mit hydrophilen Eigenschaften, eine Aktivkohle oder ein Kohlenstoffmolsieb eingesetzt und der Adsorber enthält ein oder mehrere Übergangsselemente, elementarer oder kationischer Form, aus den Gruppen VIII und/oder I B des Periodensystems. Beispiele für einsetzbare Kleselgele sind Siliciumdioxid, für einsetzbare Aluminiumoxide sind Böhmit, gamma-, delta-, theta-, kappa-, chi- und alpha-Aluminiumoxid, für einsetzbare Aktivkohlen sind Kohlen hergestellt aus Holz, Torf, Kokosnussschalen, oder auch synthetische Kohlen und Ruße, hergestellt etwa aus Erdgas, Erdöl bzw. Folgeprodukten, oder polymeren organischen Materialien, die auch Heteroatome wie z.B. Stickstoff enthalten können, und für einsetzbare Kohlenstoffmolsiebe sind Molsiebe hergestellt aus Anthrazit und "hard coal" durch partielle Oxidation, und befinden sich z.B. beschrieben in der Electronic Version of Sixth Edition of Ullmann’s Encyclopedia of Industrial Chemistry, 2000, Chapter Adsorption, Paragraph ‚Adsorbents’.

Wird der Adsorber als Formkörper, etwa für ein Festbettverfahren hergestellt, kann er in jeder beliebigen Form verwendet werden. Typische Formkörper sind Kugeln, Stränge, Hohlstränge, Sternstränge, Tabletten, Splitt, etc. mit charakteristischen Durchmessern von 0,5 bis 5 mm, oder auch Monolithe und ähnliche strukturierte Packungen (vgl. Ullmann’s Encyclopedia, Sixth Edition, 2000 Electronic Release, Chapter Fixed-Bed Reactors, Par. 2: Catalyst Forms for Fixed-Bed Reactors).

Bei der Suspensionsfahrweise wird der Adsorber in Pulverform eingesetzt. Typische Partikelgrößen in solchen Pulvern liegen bei 1 - 100 μm, es können aber auch Partikel deutlich kleiner als 1 μm verwendet werden, etwa beim Einsatz von Ruß. Die Filtration kann in Suspensionsverfahren diskontinuierlich, etwa durch Tiefenfiltration durchgeführt werden. In kontinuierlichen Verfahren bietet sich etwa die Querstromfiltration an.

Bevorzugt werden als Adsorber Zeolithe, insbesondere Zeolithe aus der Gruppe natürliche Zeolithe, Faujasit, X-Zeolith, Y-Zeolith, A-Zeolith, L-Zeolith, ZSM 5-Zeolith, ZSM 8-Zeolith, ZSM 11-Zeolith, ZSM 12-Zeolithe, Mordenit, beta-Zeolith, Pentasil-Zeolith, und Mischungen hiervon, die ionen-austauschbare Kationen aufweisen, eingesetzt.

Solche, auch kommerzielle, Zeolithe sind beschrieben in Kirk-Othmer Encyclopedia of Chemical Engineering 4th Ed. Vol 16. Wiley, NY, 1995, und auch z.B. in Catalysis and Zeolites, J. Weitkamp and L. Puppe, Eds, Springer, Berlin (1999), aufgeführt.

Es können auch sogenannte Metal Organic Frameworks (MOFs) eingesetzt werden (z.B. Li et al., Nature, 402, 1999, Seiten 276-279).

Die Kationen des Zeoliths, z.B. $H^+$ bei einem Zeolith in der H-Form oder $Na^+$ bei einem Zeolith in der Na-Form, werden bevorzugt ganz oder teilweise gegen Metallkationen, insbesondere Übergangsmetallkationen, ausgetauscht. (Beladen der Zeolithe mit Metallkationen).

Das kann z.B. durch Ionenaustausch, Tränkung oder Verdampfung von lösbaren Salzen erfolgen. Bevorzugt werden die Metalle aber durch Ionenaustausch auf den Zeolithen aufgebracht, da sie dann, wie erfindungsgemäß erkannt, eine besonders hohe Dispersion und damit eine besonders hohe Schwefel-Adsorptionskapazität aufweisen. Der Kationenaustausch ist z.B. möglich ausgehend von Zeolithen in der Alkalimetall-, H-, oder Ammonium-Form. In Catalysis and Zeolites, J. Weitkamp and L. Puppe, Eds., Springer, Berlin (1999), sind solche Ionenaustauschtechniken für Zeolithe ausführlich beschrieben.

Bevorzugte Zeolithe weisen ein Modul (molares $SiO_2 : Al_2O_3$ - Verhältnis) im Bereich von 2 bis 1000, besonders 2 bis 100, auf.

Ganz besonders werden im erfindungsgemäßen Verfahren Adsorber, insbesondere Zeolithe, eingesetzt, die ein oder mehrere Übergangsmetalle, in elementarer oder kationischer Form, aus den Gruppen VIII und IB des Periodensystems, wie Fe, Co, Ni, Ru, Rh, Pd, Os, Ir, Pt, Cu, Ag und/oder Au, bevorzugt Ag und/oder Cu, enthalten.

Der Adsorber enthält bevorzugt 0,1 bis 75 Gew.-%, insbesondere 1 bis 60 Gew.-%, besonders 2 bis 50 Gew.-%, ganz besonders 5 bis 30 Gew.-%, (jeweils bezogen auf die Gesamtmasse des Adsorbers) des Metalls bzw. der Metalle, insbesondere des Übergangsmetalls bzw. der Übergangsmetalle.

Verfahren zur Herstellung solcher metallhaltigen Adsorber sind dem Fachmann z.B. aus Larsen et al., J. Chem. Phys. 98, 1994 Seiten 11533-11540 und J. Mol. Catalysis A, 21 (2003) Seiten 237-246, bekannt.

In Catalysis and Zeolites, J. Weitkamp and L. Puppe, Eds, Springer, Berlin (1999) sind Ionenaustauschtechniken für Zeolithe ausführlich beschrieben.

Beispielsweise beschreibt A.J. Hernandez-Maldonado et al. in Ind. Eng. Chem. Res. 42, 2003, Seiten 123-29, eine geeignete Methode, in der ein Ag-Y-Zeolith hergestellt wird, durch Ionenaustausch von Na-Y-Zeolith mit einem Überschuss an Silbernitrat in wässriger Lösung (0,2 molar) bei Raumtemperatur in 24-48 Stunden. Nach dem Ionenaustausch wird der Feststoff durch Filtration isoliert, mit großen Mengen an deionisiertem Wasser gewaschen und bei Raumtemperatur getrocknet.

Beispielsweise ist auch in T.R. Felthouse et al., J. of Catalysis 98, Seiten 411-33 (1986), beschrieben, wie aus den H-Formen von Y-Zeolith, Mordenit und ZSM-5 jeweils die Pt-haltigen Zeolithe hergestellt werden.

Auch die in WO-A2-03/020850 offenbarten Methoden zur Herstellung von Cu-Y- und Ag-Y-Zeolithen durch

Ionenaustausch ausgehend von Na-Y-Zeolithen sind geeignet, um für das erfindungsgemäße Verfahren bevorzugte Adsorber zu erhalten.

Ganz bevorzugte Adsorber sind:

Ag-X-Zeolith mit einem Ag-Gehalt von 10 bis 50 Gew.-% (bezogen auf die Gesamtmasse des Adsorbers) und
Cu-X-Zeolith mit einem Cu-Gehalt von 10 bis 50 Gew.-% (bezogen auf die Gesamtmasse des Adsorbers).

Zur Durchführung der erfindungsgemäßen Vorbehandlung des Ethanols wird der Adsorber mit dem Ethanol im Allgemeinen bei Temperaturen im Bereich von 0°C bis 200°C, insbesondere von 10°C bis 50°C, in Kontakt gebracht.

Das In-Kontakt-Bringen mit dem Adsorber erfolgt bevorzugt bei einem Absolutdruck im Bereich von 1 bis 200 bar, insbesondere 1 bis 5 bar.

Besonders bevorzugt wird bei Raumtemperatur und drucklos (Atmosphärendruck) gearbeitet.

In einer bevorzugten Ausführungsform des erfindungsgemäßen Verfahrens wird das Ethanol in flüssiger Phase, d.h. in flüssiger Form oder gelöst oder suspendiert in einem Lösungsmittel oder Verdünnungsmittel, mit dem Adsorber in Kontakt gebracht.

Als Lösungsmittel kommen insbesondere solche in Betracht, die das zu reinigende Ethanol möglichst vollständig zu lösen vermögen oder sich mit diesem vollständig mischen und die unter den Verfahrensbedingen inert sind.

Beispiele für geeignete Lösungsmittel sind Wasser, cyclische und alicyclische Ether, z.B. Tetrahydrofuran, Dioxan, Methyl-tert.-butylether, Dimethoxyethan, Dimethoxypropan, Dimethyldiethylenglykol, aliphatische Alkohole wie Methanol, Ethanol, n- oder Isopropanol, n-, 2-, iso- oder tert.-Butanol, Carbonsäureester wie Essigsäuremethylester, Essigsäureethylester, Essigsäurepropylester oder Essigsäurebutylester, sowie aliphatische Etheralkohole wie Methoxypropanol.

Die Konzentration an zu reinigendem Ethanol in der flüssigen, lösungsmittelhaltigen Phase kann grundsätzlich frei gewählt werden und liegt häufig im Bereich von 20 bis 95 Gew.-%, bezogen auf das Gesamtgewicht der Lösung/ Mischung.

Eine Variante des erfindungsgemäßen Verfahrens besteht darin, dass es, drucklos oder unter Druck, in Gegenwart von Wasserstoff durchgeführt wird.

Das Verfahren kann in der Gas- oder Flüssigphase, Festbett- oder Suspensionsfahrweise durchgeführt werden, mit oder ohne Rückvermischung, kontinuierlich oder diskontinuierlich entsprechend den dem Fachmann bekannten Verfahren (z.B. beschrieben in Ullmann's Encyclopedia, sixth edition, 2000 electronic release, Chapter "Adsorption").

Um einen möglichst hohen Abreicherungsgrad der Schwefelverbindung zu erhalten, bieten sich insbesondere Verfahren mit einem geringen Grad an Rückvermischung an.

Das erfindungsgemäße Verfahren ermöglicht insbesondere die Abreicherung von Schwefel und/oder schwefelhaltigen Verbindungen aus dem Ethanol um ≥ 90, besonders ≥ 95, ganz besonders ≥ 98 Gew.-% (jeweils berechnet S).

Das erfindungsgemäße Verfahren ermöglicht insbesondere die Abreicherung von Schwefel und/oder schwefelhaltigen Verbindungen aus dem Ethanol auf einen Restgehalt von < 2, besonders < 1, ganz besonders von 0 bis < 0,1 Gew.-ppm (jeweils berechnet S), z.B. bestimmt nach Wickbold (DIN EN 41).

Mit der erfindungsgemäßen Vorbehandlung des Ethanols mit einem Adsorber wird bevorzugt ein Ethanol erhalten, das
einen Gehalt an Schwefel und/oder schwefelhaltigen organischen Verbindungen im Bereich von 0 bis 2 Gew.-ppm, besonders 0 bis 1 ppm, ganz besonders 0 bis 0,1 ppm, (jeweils berechnet S), z.B. bestimmt nach Wickbold (DIN EN 41),

einen Gehalt an $C_{3-4}$-Alkanolen im Bereich von 1 bis 5000 Gew.-ppm, besonders 5 bis 3000 Gew.-ppm, ganz besonders 10 bis 2000 Gew.-ppm,
einen Gehalt an Methanol im Bereich von 1 bis 5000 Gew.-ppm, besonders 5 bis 3000 Gew.-ppm, ganz besonders 10 bis 2000 Gew.-ppm,
einen Gehalt an Ethylacetat im Bereich von 1 bis 5000 Gew.-ppm, besonders 5 bis 3000 Gew.-ppm, ganz besonders 10 bis 2000 Gew.-ppm, und
einen Gehalt an 3-Methyl-butanol-1 im Bereich von 1 bis 5000 Gew.-ppm, besonders 5 bis 3000 Gew.-ppm, ganz besonders 10 bis 2000 Gew.-ppm,
aufweist.

Der Gehalt an $C_{3-4}$-Alkanolen, Methanol, Ethylacetat und 3-Methyl-butanol-1 wird z.B. bestimmt mittels Gaschromatographie (30 m DB-WAX-Säule, Innendurchmesser: 0,32 mm, Filmdicke: 0,25 μm, FID-Detektor, Temperaturprogramm: 35°C (5 Min.), 10°C/Min. Aufheizrate, 200°C (8 Min.).

Das erfindungsgemäß bevorzugt eingesetzte Bio-Ethanol wird im Allgemeinen aus Agrarprodukten wie Melasse, Rohrzuckersaft, Maisstärke oder aus Produkten der Holzverzuckerung und aus Sulfitablaugen durch Fermentation erzeugt.

Bevorzugt wird Bio-Ethanol eingesetzt, dass durch Fermentation von Glukose unter $CO_2$-Abspaltung erhalten wurde (K. Weissermel und H.-J. Arpe, Industrial Organic Chemistry, Wiley-VCH, Weinheim, 2003, p. 194; Electronic

Version of Sixth Edition of Ullmann's Encyclopedia of Industrial Chemistry, 2000, Chapter Ethanol, Paragraph Fermentation). Das Ethanol wird in der Regel durch Destillationsverfahren aus den Fermentationsbrühen gewonnen: Electronic Version of Sixth Edition of Ullmann's Encyclopedia of Industrial Chemistry, 2000, Chapter Ethanol, Paragraph Recovery and Purification.

Verfahren zur Herstellung eines Ethylamins durch Umsetzung von Ethanol mit Ammoniak, einem primären Amin oder einem sekundären Amin sind aus der Literatur bekannt, z.B. aus Ullmann's Encyclopedia of Industrial Chemistry, 6th Edition, 2000 electronic release, ‚aliphatic Amines: Production from alcohols'.

Typische Katalysatoren enthalten Cu, Co, Ni und/oder Fe, sowie häufig auch Edelmetalle wie Ru, Pt, Pd sowie Re. Die Katalysatoren können dotiert sein, etwa mit Ag, Zn, In, Mn, Alkalimetallen und/oder Mo.

Als Trägermaterial für diese Aktivmetalle werden häufig Aluminiumoxid (gamma, delta, theta, alpha, kappa, chi oder Mischungen daraus), Siliziumoxid, Zeolithe, Alumosilicate, etc. sowie Mischungen aus diesen Trägern verwendet.

Die Katalysatoren können nach bekannten Verfahren, z.B. durch Fällung, Auffällung, Imprägnierung, hergestellt werden.

Besonders bevorzugte Heterogenkatalysatoren für die Aminierung des erfindungsgemäß vorbehandelten Ethanols enthalten in ihrer katalytisch aktiven Masse vor der Behandlung mit Wasserstoff

20 bis 85 Gew.-%, bevorzugt 20 bis 65 Gew.-%, besonders bevorzugt 22 bis 40 Gew.%, $Al_2O_3$, $TiO_2$, $ZrO_2$ und/oder $SiO_2$,

1 bis 30 Gew.-%, besonders bevorzugt 2 bis 25 Gew.-%, sauerstoffhaltige Verbindungen des Kupfers, berechnet als CuO, und

14 bis 70 Gew.-%, bevorzugt 15 bis 50 Gew.%, besonders bevorzugt 21 bis 45 Gew.-%, sauerstoffhaltige Verbindungen des Nickels, berechnet als NiO, wobei bevorzugt das Molverhältnis von Nickel zu Kupfer größer 1, insbesondere größer 1,2, ganz besonders 1,8 bis 8,5, ist,

In einer weiteren Variante enthalten diese besonders bevorzugten Katalysatoren zusätzlich in ihrer katalytisch aktiven Masse vor der Behandlung mit Wasserstoff
15 bis 50 Gew.-%, besonders bevorzugt 21 bis 45 Gew.-%, sauerstoffhaltige Verbindungen des Kobalts, berechnet als CoO.

Die sauerstoffhaltigen Verbindungen des Kupfers, Nickels und gegebenenfalls Kobalts, jeweils berechnet als CuO, NiO und CoO, der bevorzugten Katalysatoren sind im allgemeinen insgesamt in Mengen von 15 bis 80 Gew.-%, bevorzugt 35 bis 80 Gew.-%, besonders bevorzugt 60 bis 78 Gew.-%, in der katalytisch aktiven Masse (vor der Behandlung mit Wasserstoff) enthalten, wobei besonders bevorzugt das Molverhältnis von Nickel zu Kupfer größer 1 ist.

Weitere bevorzugte Heterogenkatalysatoren im erfindungsgemäßen Verfahren sind

in DE-A-19 53 263 (BASF AG) offenbarte Katalysatoren enthaltend Kobalt, Nickel und Kupfer und Aluminiumoxid und/oder Siliciumdioxid mit einem Metallgehalt von 5 bis 80 Gew.-%, insbesondere 10 bis 30 Gew.-%, bezogen auf den gesamten Katalysator, wobei die Katalysatoren, berechnet auf den Metallgehalt, 70 bis 95 Gew.-% einer Mischung aus Kobalt und Nickel und 5 bis 30 Gew.-% Kupfer enthalten und wobei das Gewichtsverhältnis von Kobalt zu Nickel 4 : 1 bis 1 : 4, insbesondere 2 : 1 bis 1 : 2, beträgt, beispielsweise der in den dortigen Beispielen verwendete Katalysator mit der Zusammensetzung 10 Gew.-% CoO, 10 Gew.-% NiO und 4 Gew.-% CuO auf $Al_2O_3$, in EP-A-382 049 (BASF AG) offenbarte Katalysatoren, deren katalytisch aktive Masse vor der Behandlung mit Wasserstoff
20 bis 85 Gew.-%, bevorzugt 70 bis 80 Gew.-%, $ZrO_2$,
1 bis 30 Gew.-%, bevorzugt 1 bis 10 Gew.%, CuO,
und jeweils 1 bis 40 Gew.-%, bevorzugt 5 bis 20 Gew.-%, CoO und NiO enthält, beispielsweise die in loc. cit. auf Seite 6 beschriebenen Katalysatoren mit der Zusammensetzung 76 Gew.% Zr, berechnet als $ZrO_2$ 4 Gew.-% Cu, berechnet als CuO, 10 Gew.-% Co, berechnet als CoO, und 10 Gew.-% Ni, berechnet als NiO,
in EP-A-963 975 (BASF AG) offenbarte Katalysatoren, deren katalytisch aktive Masse vor der Behandlung mit Wasserstoff
22 bis 40 Gew.-% $ZrO_2$,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, wobei das molare Ni : Cu-Verhältnis größer 1 ist,
15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als $Al_2O_3$ bzw. $MnO_2$
und keine sauerstoffhaltigen Verbindungen des Molybdäns enthält,
beispielsweise der in loc. cit., Seite 17, offenbarte Katalysator A mit der Zusammensetzung 33 Gew.-% Zr, berechnet

als $ZrO_2$, 28 Gew.-% Ni, berechnet als NiO, 11 Gew.-% Cu, berechnet als CuO und 28 Gew.-% Co, berechnet als CoO, in EP-A-696 572 (BASF AG) offenbarte Katalysatoren, deren katalytisch aktive Masse vor der Reduktion mit Wasserstoff 20 bis 85 Gew.-% $ZrO_2$, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als $MoO_3$ und 0 bis 10 Gew.-% sauerstoffhaltige Verbindungen des Aluminiums und/ oder Mangans, berechnet als $Al_2O_3$ bzw. $MnO_2$ enthält, beispielsweise der in loc. cit., Seite 8, offenbarte Katalysator mit der Zusammensetzung 31,5 Gew.-% $ZrO_2$, 50 Gew.-% NiO, 17 Gew.-% CuO und 1,5 Gew.-% $MoO_3$,
in EP A1-1 270 543 (BASF AG) beschriebene Katalysatoren, enthaltend mindestens ein Element oder eine Verbindung eines Elementes aus den Gruppen VIII und IB des Periodensystems,
und
in der deutsche Patentanmeldung Nr. 10261195.5 vom 20.12.02 (BASF AG) beschriebene Katalysatoren, bei deren Herstellung eine Fällung von katalytisch aktiven Komponenten auf monoklines, tetragonales oder kubisches Zirkoniumdioxid erfolgte.
Das erfindungsgemäße Verfahren eignet sich beispielsweise zur Herstellung von Ethylaminen des Formel 1

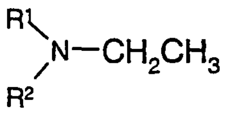
(I),

in der

| | |
|---|---|
| $R^1$, $R^2$ | Wasserstoff (H), Alkyl, wie $C_{1-200}$-Alkyl, Cycloalkyl, wie $C_{3-12}$-Cycloalkyl, Hydro- xyalkyl, wie $C_{1-20}$-Hydroxyalkyl, Aminoalkyl, wie $C_{1-20}$-Aminoalkyl, Hydroxyal- kylaminoalkyl, wie $C_{2-20}$-Hydroxyalkylaminoalkyl, Alkoxyalkyl, wie $C_{2-30}$- Alkoxyalkyl, Dialkylaminoalkyl, wie $C_{3-30}$-Dialkylaminoalkyl, Alkylaminoalkyl, wie $C_{2-30}$-Alkylaminoalkyl, Aryl, Heteroaryl, Aralkyl, wie $C_{7-20}$-Aralkyl, und Alkyl- aryl, wie $C_{7-20}$-Alkylaryl, oder gemeinsam $-(CH_2)_j-X-(CH_2)_k-$, |
| X | $CH_2$, $CHR^3$, Sauerstoff (O), Schwefel (S) oder $NR^3$, |
| $R^3$ | Wasserstoff (H), Alkyl, wie $C_{1-4}$-Alkyl, Alkylphenyl, wie $C_{7-40}$-Alkylphenyl, und |
| j, k | eine ganze Zahl von 1 bis 4 |

bedeuten.
Das erfindungsgemäße Verfahren findet daher bevorzugt zur Herstellung eines Ethylamins I Anwendung, indem man das erfindungsgemäß vorbehandelte Bio-Ethanol mit einer Stickstoffverbindung der Formel II

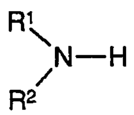
(II),

wobei $R^1$ und $R^2$ die oben genannten Bedeutungen haben, umsetzt.
Zur Herstellung des Ethylamins I wird demnach rein formal ein Wasserstoffatom der Stickstoffverbindung II durch den Rest $CH_3CH_2$- unter Freisetzung von einem Moläquivalent Wasser ersetzt.
Die Substituenten $R^1$ bis $R^3$, die Variable X und die Indizes j, k in den Verbindungen I und II haben unabhängig voneinander folgende Bedeutungen:

$R^1$, $R^2$:

- Wasserstoff (H),
- Alkyl, wie $C_{1-200}$-Alkyl, bevorzugt $C_{1-20}$-Alkyl, besonders bevorzugt $C_{1-14}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, isoHexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, insbesondere $C_{1-4}$-Alkyl,

- Cycloalkyl, wie $C_{3-12}$-Cycloalkyl, bevorzugt $C_{3-8}$-Cycloalkyl, wie Cyclopropyl, Cyclobutyl, Cyclopentyl, Cyclohexyl, Cycloheptyl und Cyclooctyl, besonders bevorzugt Cyclopentyl und Cyclohexyl,

- Hydroxyalkyl, wie $C_{1-20}$-Hydroxyalkyl, bevorzugt $C_{1-8}$-Hydroxyalkyl, besonders bevorzugt $C_{1-4}$-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-(Hydroxymethyl)ethyl,

- Aminoalkyl, wie $C_{1-20}$-Aminoalkyl, bevorzugt $C_{1-8}$-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Aminoethyl)aminomethyl,

- Hydroxyalkylaminoalkyl, wie $C_{2-20}$-Hydroxyalkylaminoalkyl, bevorzugt $C_{3-8}$-Hydroxyalkylaminoalkyl, wie (2-Hydroxyethylamino)methyl, 2-(2-Hydroxyethylamino)ethyl und 3-(2-Hydroxyethylamino)propyl,

- Alkoxyalkyl, wie $C_{2-30}$-Alkoxyalkyl, bevorzugt $C_{2-20}$-Alkoxyalkyl, besonders bevorzugt $C_{2-8}$-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt $C_{2-4}$-Alkoxyalkyl,

- Dialkylaminoalkyl, wie $C_{3-30}$-Dialkylaminoalkyl, bevorzugt $C_{3-20}$-Dialkylaminoalkyl, besonders bevorzugt $C_{3-10}$-N,N-Dialkylaminoalkyl, wie (N,N-Dimethylamino)-methyl, (N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl, 2-(N,N-Di-iso-propylamino)ethyl, $(R^3)_2N-(CH_2)_q$ (q = 1 bis 6), ganz besonders 3-(N,N-Dimethylamino)propyl

- Alkylaminoalkyl, wie $C_{2-30}$-Alkylaminoalkyl, bevorzugt $C_{2-20}$-Alkylaminoalkyl, besonders bevorzugt $C_{2-8}$-Alkylaminoalkyl, wie Methylaminomethyl, 2-(Methylamino)ethyl, Ethylaminomethyl, 2-(Ethylamino)ethyl und 2-(iso-Propylamino)-ethyl, $(R^3)HN-(CH_2)_q$ (q = 1 bis 6),

- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,

- Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,

- Aralkyl, wie $C_{7-20}$-Aralkyl, bevorzugt $C_{7-12}$-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,

- Alkylaryl, wie $C_{7-20}$-Alkylaryl, bevorzugt $C_{7-12}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,

- oder beide Reste bedeuten gemeinsam eine $-(CH_2)_j-X-(CH_2)_k-$ Gruppe, wie - $(CH_2)_3$-, $-(CH_2)_4$-, $-(CH_2)_5$-, $-(CH_2)_6$-, $-(CH_2)_7$-, $-(CH_2)-O-(CH_2)_2$-, $-(CH_2)-NR^3-(CH_2)_2$-, $-(CH_2)-CHR^3-(CH_2)_2$-, $-(CH_2)_2-O-(CH_2)_2$-, $-(CH_2)_2-NR^3-(CH_2)_2$-, $-(CH_2)_2-CHR^3-(CH_2)_2$-, $-CH_2-O-(CH_2)_3$-, $-CH_2-NR^3-(CH_2)_3$-,

$R^3$:

- Wasserstoff (H),

- Alkyl, besonders $C_{1-4}$-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,

- Alkylphenyl, besonders $C_{7-40}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl,

X:

- $CH_2$, $CHR^3$, Sauerstoff (O), Schwefel (S) oder $NR^3$, bevorzugt $CH_2$, NH und O,

j:

- eine ganze Zahl von 1 bis 4 (1, 2, 3 oder 4), bevorzugt 1 und 2, und

k:

- eine ganze Zahl von 1 bis 4 (1, 2, 3 oder 4), bevorzugt 1 und 2.

Als Aminierungsmittel bei der hydrierenden Aminierung des Bio-Ethanols in Gegenwart von Wasserstoff können sowohl Ammoniak als auch primäre oder sekundäre, aliphatische oder cycloaliphatische oder aromatische Amine eingesetzt werden.

Bei Verwendung von Ammoniak als Aminierungsmittel wird die alkoholische Hydroxylgruppe zunächst in die primäre Aminogruppen ($-NH_2$) umgewandelt. Das so gebildete primäre Ethylamin kann mit weiterem Ethanol zu dem entsprechenden sekundären Amin (Diethylamin) und dieses wiederum mit weiterem Alkohol zu dem entsprechenden tertiären Amin (Triethylamin) reagieren. Je nach Zusammensetzung des Reaktionsansatzes oder des Eduktstroms (bei kontinuierlicher Fahrweise) und je nach den angewandten Reaktionsbedingungen - Druck, Temperatur, Katalysator, Reaktionszeit (Katalysatorbelastung) - lassen sich auf diese Weise je nach Wunsch bevorzugt primäre, sekundäre oder tertiäre Ethylamine darstellen.

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie Ethyldiisopropylamin und Ethyldicyclohexylamin verwendet.

Beispielsweise werden die folgenden Mono- und Dialkylamine als Aminierungsmittel verwendet: Methylamin, Dimethylamin, Ethylamin, Diethylamin, n-Propylamin, Di-n-propyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin.

Mit dem erfindungsgemäßen Verfahren besonders bevorzugt hergestellte Amine sind zum Beispiel Monoethylamin (aus Ethanol und Ammoniak), Diethylamin (aus Ethanol und Monoethylamin), Triethylamin (aus Ethanol und Diethylamin), Mono-/Di-/Triethylamin-Gemisch (aus Ethanol und Ammoniak) und Dimethylethylamin (aus Ethanol und Dimethylamin).

Das Aminierungsmittel kann bezüglich der zu aminierenden alkoholischen Hydroxylgruppe in stöchiometrischen, unter- oder überstöchiometrischen Mengen eingesetzt werden.

Bevorzugt wird im Falle der Aminierung mit primären oder sekundären Aminen das Amin in ca. stöchiometrischer Menge oder geringfügig überstöchiometrischer Menge pro Mol alkoholischer Hydroxylgruppe eingesetzt.

Speziell Ammoniak wird im allgemeinen mit einem 1,5 bis 250-fachen, bevorzugt 2 bis 100-fachen, insbesondere 2 bis 10-fachen molaren Überschuss pro Mol umzusetzender alkoholischer Hydroxylgruppe eingesetzt.

Höhere Überschüsse sowohl an Ammoniak als auch an primären oder sekundären Aminen sind möglich.

Das erfindungsgemäße Verfahren lässt sich diskontinuierlich oder bevorzugt kontinuierlich wie folgt durchführen, wobei der Katalysator bevorzugt als Festbett im Reaktor angeordnet ist.

Die Ausführungsform als Wirbelbettreaktion mit in auf- und abwirbelnder Bewegung befindlichem Katalysatormaterial ist jedoch ebenfalls möglich.

Die Aminierung kann in der Flüssigphase oder in der Gasphase durchgeführt werden. Bevorzugt ist das Festbettverfahren in der Gasphase.

Beim Arbeiten in der Flüssigphase leitet man den die Edukte (Alkohol plus Ammoniak oder Amin) simultan in flüssiger Phase bei Drücken von im allgemeinen 5 bis 30 MPa (50-300 bar), bevorzugt 5 bis 25 MPa, besonders bevorzugt 15 bis 25 MPa, und Temperaturen von im allgemeinen 80 bis 300°C, bevorzugt 120 bis 270°C, besonders bevorzugt 130 bis 250°C, insbesondere 170 bis 230°C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Katalysatorbelastung liegt im allgemeinen im Bereich von 0,05 bis 5, bevorzugt 0,1 bis 2, besonders bevorzugt 0,2 bis 0,6, kg Alkohol pro Liter Katalysator (Schüttvolumen) und Stunde. Gegebenenfalls kann eine Verdünnung der Edukte mit einem geeigneten Lösungsmittel, wie Tetrahydrofuran, Dioxan, N-Methylpyrrolidon oder

Ethylenglykoldimethylether, erfolgen. Es ist zweckmäßig, die Reaktanden bereits vor der Zuführung in das Reaktionsgefäß zu erwärmen, und zwar bevorzugt auf die Reaktionstemperatur.

Beim Arbeiten in der Gasphase werden die gasförmigen Edukte (Alkohol plus Ammoniak oder Amin) in einem zur Verdampfung ausreichend groß gewählten Gasstrom, bevorzugt Wasserstoff, bei Drücken von im allgemeinen 0,1 bis 40 MPa (1 bis 400 bar), bevorzugt 0,1 bis 10 MPa, besonders bevorzugt 0,1 bis 7 MPa, in Gegenwart von Wasserstoff über den Katalysator geleitet. Die Temperaturen für die Aminierung betragen im allgemeinen 80 bis 300°C, bevorzugt 120 bis 270°C, besonders bevorzugt 160 bis 250°C. Es ist dabei sowohl eine Anströmung des Katalysatorfestbetts von oben als auch von unten möglich. Den erforderlichen Gasstrom erhält man bevorzugt durch eine Kreisgasfahrweise.

Die Katalysatorbelastung liegt im allgemeinen im Bereich von 0,01 bis 2, bevorzugt 0,05 bis 0,5, kg Alkohol pro Liter Katalysator (Schüttvolumen) und Stunde.

Der Wasserstoff wird der Reaktion im allgemeinen in einer Menge von 5 bis 400 l, bevorzugt in einer Menge von 50 bis 200 l pro Mol Alkoholkomponente zugeführt, wobei die Literangaben jeweils auf Normalbedingungen umgerechnet wurden (S.T.P.). Sowohl beim Arbeiten in der Flüssigphase als auch beim Arbeiten in der Gasphase ist die Anwendung höherer Temperaturen und höherer Gesamtdrücke möglich. Der Druck im Reaktionsgefäß, welcher sich aus der Summe der Partialdrücke des Aminierungsmittels, des Alkohols und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe) wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z.B. destillativ.

Aus dem Reaktionsaustrag werden, nachdem dieser zweckmäßigerweise entspannt worden ist, das überschüssige Aminierungsmittel und der Wasserstoff entfernt und die erhaltenen Aminierungsprodukte (Ethylamine) durch Destillation bzw. Rektifikation, Flüssigextraktion oder Kristallisation gereinigt. Das überschüssige Aminierungsmittel und der Wasserstoff werden vorteilhaft wieder in die Reaktionszone zurückgeführt. Das gleiche gilt für die eventuell nicht vollständig umgesetzten Alkohol.

Die unter Anwendung des erfindungsgemäßen Verfahrens hergestellten Amine eignen sich u. a. als Zwischenprodukte bei der Herstellung von Kraftstoffadditiven (US-A-3,275,554; DE-A-21 25 039 und DE-A-36 11 230), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

## Beispiele

### I) Aminierung von Bio-Ethanol und erfindungsgemäß vorbehandeltem Bio-Ethanol mit Ammoniak

Beim Einsatz von Bio-Ethanol mit 0,5 - 2 ppm Schwefelgehalt zur Synthese von Ethylaminen (Mono-, Di- und Triethylamin) durch Umsetzung mit Ammoniak bei 150-250°C und 60 bar an einem Aminierungs-Katalysator (3,4 Gew.-% Cu, 8,3 Gew.-% Co, 8,3 Gew.-% Ni geträgert auf gamma-$Al_2O_3$) wurde eine deutlich schnellere Katalysatordesaktivierung festgestellt, als dies bei Einsatz von synthetischem Ethanol mit < 0,1 ppm Schwefelgehalt der Fall ist.

Auf Ausbauproben des desaktivierten Synthesekatalysators nach Einsatz von Bio-Ethanol wurde ein deutlich erhöhter Schwefelgehalt gemessen:

| | frisch | Ausbau |
|---|---|---|
| S-Gehalt / ppm | 25 | 670 |

Die Bestimmung des S-Gehalts im Ein- und Austrag erfolgte (in allen Beispielen) coulometrisch (DIN 51400 Teil 7) mit einer Nachweisgrenze von 2 ppm.

In dem bei der Aminierung verwendeten Bio-Ethanol-Feed wurden 0,5-2 ppm Schwefel detektiert. Genauere Analysen ergaben, dass eine typische organische Schwefelverbindung im Bio-Ethanol Dimethylsulfid ist. Daneben sind

Sulfat-Schwefelspezies sowie gegebenenfalls weitere organische Schwefelspezies, wie z.B. $C_{2-10}$-Dialkylsulfide, $C_{2-10}$-Dialkylsulfoxide, 3-Methylthio-1-propanol und/oder S-haltige Aminosäuren, im Bio-Ethanol enthalten.

Das Beispiel zeigt, dass die im Bio-Ethanol enthaltenen Schwefel-Verbindungen, insbesondere die organischen Schwefelverbindungen, insbesondere Dimethylsulfid, zu einer beschleunigten Katalysatordesaktivierung geführt haben.

II) Erfindungsgemäße Behandlung von Bio-Ethanol durch In-Kontakt-Bringen mit einem Adsorber

Herstellung von Ag-Zeolithen

Beispiel 1: Pulver-Ag-Zeolith

Eine $AgNO_3$-Lösung (7,71 g $AgNO_3$ in Wasser, 200 ml gesamt) wurde in einem Becherglas vorgelegt, der Zeolith (ZSM-5, 200 g, molares $SiO_2/Al_2O_3$-Verhältnis = 40-48, Na-Form) unter Rühren langsam dazu gegeben und bei Raumtemperatur 2 h gerührt. Dann wurde der Adsorber über einen Faltenfilter filtriert. Danach wurde der Adsorber 16 h bei 120°C im dunklen Trockenschrank getrocknet. Der Adsorber enthielt 2,1 Gew.-% Ag (bezogen auf die Gesamtmasse der Adsorbers).

Beispiel 2: Formkörper-Ag-Zeolith

Eine $AgNO_3$-Lösung (22,4 g in Wasser, 100 ml gesamt) wurde in einem Becherglas vorgelegt. Der Zeolith (65 g Molsieb 13X in Form von Kugeln mit 2,7 mm Durchmesser, molares $SiO_2/Al_2O_3$-Verhältnis = 2, Na-Form) wurde in der Apparatur vorgelegt. Nun wurden 400 ml Wasser eingefüllt und bei Raumtemperatur in einer kontinuierlichen Anlage im Kreislauf gepumpt. Die Ag-Nitrat-Lösung wurde in 1 h zugetropft. Nun wurde über Nacht (23 h) im Kreislauf gepumpt. Danach wurde der Adsorber mit 12 Liter vollentsalztem Wasser Nitrat-frei abgewaschen und anschließend bei 120°C über Nacht im dunklen Trockenschrank getrocknet. Der Adsorber enthielt 15,9 Gew.-% Ag (bezogen auf die Gesamtmasse der Adsorbers).

Beispiele A

Alle ppm-Angaben in diesem Dokument beziehen sich auf das Gewicht.

Zur Testung der Entschwefelung wurde jeweils 10 g des Adsorbers (vgl. die folgende Tabelle) in einem Trockenschrank bei 150°C über Nacht zur Entfernung adsorbierten Wassers ausgeheizt. Nach dem Erkalten des Feststoffs wurde er aus dem Trockenschrank entnommen und mit 300 ml Ethanol (Ethanol absolut, > 99,8 %, Quelle: Riedel de Haën) übergossen. Dem Ethanol waren ca. 17 ppm Dimethylsulfid (entspricht ca. 9 ppm Schwefel) zugegeben, da in Vorversuchen gefunden wurde, dass Dimethylsulfid eine für die in Bio-Ethanol enthaltenen organischen Schwefelverbindungen repräsentative Schwefelverbindung ist.

Der Ag/ZSM-5-Adsorber wurde hergestellt durch ionenaustausch des Na-ZSM-5 mit einer wässrigen $AgNO_3$-Lösung (50 g ZSM-5, 1,94 g $AgNO_3$ 50 ml Tränklösung). Dabei wurde ein kommerziell verfügbarer ZSM-5 (molares $SiO_2/Al_2O_3$ Verhältnis = 40-48, Na-Form, ALSI-PENTA®) verwendet. Der Katalysator wurde anschließend bei 120°C getrocknet.

Der Ag/$SiO_2$-Adsorber wurde hergestellt durch Tränkung von $SiO_2$ (BET ca. 170 $m^2/g$, $Na_2O$-Gehalt: 0,4 Gew.-%) mit einer wässrigen $AgNO_3$-Lösung (40 g $SiO_2$, 1,6 g $AgNO_3$ 58 ml Tränklösung). Der Katalysator wurde anschließend bei 120°C getrocknet und bei 500°C calciniert.

Der Ag/$Al_2O_3$-Adsorber wurde hergestellt durch Tränkung von gamma-$Al_2O_3$ (BET ca. 220 $m^2/g$) mit einer wässrigen $AgNO_3$-Lösung (40 g $Al_2O_3$, 1,6 g $AgNO_3$ 40 ml Tränklösung). Der Katalysator wurde anschließend bei 120 °C getrocknet und bei 500°C calciniert.

Die Ethanol/Adsorber-Suspension wurde in einen 4-Hals-Glaskolben überführt, in den für ca. 5 Min. Stickstoff zur Inertisierung eingeleitet wurde. Anschließend wurde der Kolben verschlossen und die Suspension für 5 h bei Raumtemperatur gerührt. Nach dem Versuch wurde der Adsorber über einen Faltenfilter filtriert. Vom Filtrat und ggf. auch vom Adsorber wurde der Schwefelgehalt coulometrisch bestimmt:

| | S-Gehalte / ppm | | | |
|---|---|---|---|---|
| Adsorber | Eintrag | Austrag | Adsorber frisch | beladener Adsorber |
| Ag/ZSM-5 | 9 | < 2 | 25 | 230 |
| ZSM-5 | 9 | 4 | n.b. | n.b. |

(fortgesetzt)

| | S-Gehalte / ppm | | | |
|---|---|---|---|---|
| Adsorber | Eintrag | Austrag | Adsorber frisch | beladener Adsorber |
| $Ag/Al_2O_3$ | 9 | 2 | n.b. | n.b. |
| $Ag/SiO_2$ | 9 | 4 | n.b. | n.b. |
| (n.b. = nicht bestimmt) | | | | |

Die Tabelle zeigt, dass insbesondere der Silber-beladene Zeolith in der Lage war, den Schwefelgehalt auf Werte unterhalb der Nachweisgrenze (= 2 ppm) abzusenken.

Auch nach dreimaligem Einsatz derselben Ag/ZSM-5-Probe wurde nach Versuchsdurchführung < 2 ppm Schwefel im Ethanol detektiert.

Auch bei den Adsorbern, bei denen Silber auf andere Träger wie $Al_2O_2$ oder $SiO_2$ aufgebracht wurde, konnte eine Entschwefelung festgestellt werden. Auch der undotierte Zeolith führte zu einer gewissen Schwefelabreicherung aus dem Ethanol. Das beste Resultat wurde am silberdotierten Zeolithen erhalten.

Auch sonstige Materialien, wie $Cu/ZnO/Al_2O_3$-Katalysatoren oder Ni-Katalysatoren, waren für die S-Entfernung aus Bio-Ethanol geeignet, aber weniger gut als der Silber-dotierte Zeolith, selbst dann, wenn bei erhöhter Temperatur und unter Zugabe von Wasserstoff gearbeitet wurde.

Beispiele B

Beispiel B1

Zur Testung der Entschwefelung wurde 20 g des pulverförmigen Adsorbers Ag-ZSM5, 2,1 Gew.-% Ag verwendet (siehe Beispiel 1) und mit 300 ml Ethanol (Ethanol absolut, > 99,8 %, Quelle: Riedel de Haën) übergossen. Dem Ethanol waren ca. 175 ppm Dimethylsulfid (> 99 %, Merck) (entspricht ca. 90 ppm Schwefel) zugegeben, da in Vorversuchen gefunden wurde, dass Dimethylsulfid eine für die in Bio-Ethanol enthaltenen organischen Schwefelverbindungen repräsentative Schwefelverbindung ist. Die Ethanol/Adsorber-Suspension wurde in einen geschlossenen 4-Hals-Glaskolben überführt. Die Suspension wurde bei Raumtemperatur und Normaldruck gerührt. Nach dem Versuch wurde der Adsorber über einen Faltenfilter filtriert. Vom Eintrag, Filtrat und ggf. auch vom Adsorber wurde der Schwefelgehalt coulometrisch bestimmt. Dieselbe Ag-ZSM5-Probe wurde noch dreimal eingesetzt:

| Einsatz | Verweilzeit Stunden | Eintrag S ppm | Austrag S ppm | Beladener Adsorber S ppm |
|---|---|---|---|---|
| 1 | 5 | 84 | <2 | 1300 |
| 2 | 24 | 84 | <2 | 2800 |
| 3 | 24 | 95 | 10 | 4600 |
| 4 | 24 | 97 | 29 | 5900 |

Beispiel B2

Zur Testung der Entschwefelung wurde pulverförmige Entschwefelungsmaterialien mit 300 ml Ethanol (Ethanol absolut, > 99,8 %, Riedel de Haën) übergossen. Dem Ethanol waren ca. 175 ppm Dimethylsulfid (> 99 %, Merck) (entspricht ca. 90 ppm Schwefel) zugegeben. Die Ethanol/Adsorber-Suspension wurde in einen geschlossenen 4-Hals-Glaskolben überführt. Die Suspension wurde bei Raumtemperatur und Normaldruck für 24 Stunden gerührt. Nach dem Versuch wurde der Adsorber über einen Faltenfilter filtriert. Vom Eintrag, Filtrat und ggf. auch vom Adsorber wurde der Schwefelgehalt coulometrisch bestimmt.

| Adsorber | Adsorber Gew.-% | Eintrag S ppm | Austrag S ppm | Beladener Adsorber S ppm |
|---|---|---|---|---|
| 40 CuO/40 ZnO/20 $Al_2O_3$ in Gew.-% | 8,5 | 84 | 64 | 22 |
| 17NiO/15 $SiO_2$/ 5 $Al_2O_3$/ 5 $ZrO_2$, in Gew.-% | 8,5 | 95 | 58 | 9 |

(fortgesetzt)

| Adsorber | Adsorber Gew.-% | Eintrag S ppm | Austrag S ppm | Beladener Adsorber S ppm |
|---|---|---|---|---|
| 5 Gew.-% Pd/C | 2,5 | 100 | 39 | 2300 |
| 2. Einsatz des Pd/C-Adsorbers | | 97 | 60 | 3000 |

Die Materialien $CuO$-$ZnO/Al_2O_3$ und $NiO/SiO_2/Al_2O_3/ZrO_2$ sind für die Entschwefelung geeignet, aber weniger gut als z.B. ein Silber-dotierter Zeolith, selbst dann, wenn bei erhöhter Temperatur und unter Zugabe von Wasserstoff gearbeitet wurde. Wird Palladium auf Kohle eingesetzt, wird Schwefel aus Ethanol aufgenommen.

Beispiel B3

Zur Testung des Adsorbers wurde eine kontinuierliche Festbettanlage mit einem Gesamtvolumen von 192 ml mit 80,5 g Ag-13X Kugeln (15,9 Gew.-% Ag, 2,7 mm Kugeln, beschrieben in Beispiel 2) gefüllt. Dem Feed Ethanol (Ethanol absolut, > 99,8 %, Riedel de Haën) waren ca. 80 ppm Dimethylsulfid, (> 99 %, Merck) (entspricht ca. 40 ppm Schwefel) zugegeben. Das Feed wurde in Sumpffahrweise über den Adsorber gefahren. Während der Probennahme wurde immer die Probeflasche mit einer Eis/SalzMischung gekühlt.

| Standzeit | Kumulierte Belastung (ppm S/g Adsorber) | Eintrag S ppm | Austrag S ppm |
|---|---|---|---|
| 24 | 934 | 38 | <2 |
| 48 | 1623 | 41 | <2 |
| 72 | 2222 | 42 | <2 |

Die Schwefelbestinimung im Ein- und Austrag erfolgte (in allen Beispielen) coulometrisch (DIN 51400 Teil 7) mit einer Nachweisgrenze von 2 ppm.

Beispiel B4

Zur Testung der Entschwefelung wurde jeweils 4 g des Adsorbers (vgl. die folgende Tabelle) mit 500 ml Ethanol (Ethanol absolut, > 99,8 %, Riedel de Haën) übergossen. Dem Ethanol waren ca. 390 ppm Dimethylsulfid, (> 99 %, Merck) (entspricht ca. 200 ppm Schwefel) zugegeben.

Die Herstellung des Ag-13X ist beschrieben in Beispiel 1. CBV100 und CBV720 sind Zeolith-Y Systeme. Die Dotierung mit Metallen wurde durch Kationenaustausch analog Beispiel 1 durchgeführt, hierbei wurden $AgNO_3$- bzw. $CuNO_3$-Lösungen eingesetzt. Der Cu-CPV720 wurde anschließend bei 450 °C in $N_2$ calciniert.

Die Ethanol/Adsorber-Suspension wurde in einen 4-Hals-Glaskolben überführt und für 24 h bei Raumtemperatur drucklos gerührt. Nach dem Versuch wurde der Adsorber über einen Faltenfilter filtriert. Vom Filtrat und ggf. auch vom Adsorber wurde der Schwefelgehalt coulometrisch bestimmt:

| | | S-Gehalte / ppm | | |
|---|---|---|---|---|
| Adsorber | Form | Eintrag | Austrag | beladener Adsorber |
| Kein | - | 200 | 170 | - |
| Ag-13X | Kugeln (2,7 mm) | 200 | 96 | n.b |
| Ag-CBV100 | Pulver | 190 | 13 | 18000 |
| Ag-CBV720 | Pulver | 190 | 77 | n.b. |
| Cu-CBV720 | Pulver | 190 | 97 | 390 |
| (n.b. = nicht bestimmt) | | | | |

Die Tabelle zeigt, dass sowohl Silber-dotierte Zeolithe als auch Kupfer-dotierte Zeolithe in der Lage sind, Ethanol zu entschwefeln.

Beispiel

Verschiedene kommerzielle Bio-Ethanol Qualitäten wurden untersucht auf ihren Schwefelgehalt

| | Bio-EtOH 1 | Bio-EtOH 2 | Bio-EtOH 3 | Bio-EtOH 4 | Bio-EtOH 5 | Bio-EtOH 6 | Bio-EtOH 7 |
|---|---|---|---|---|---|---|---|
| Ges.-S (Gew.-ppm) | 0,6 | 1 | 0,6 | 8 | 2 | 49 | 2 |
| Sulfat-S (Gew.-ppm) | 0,33 | 0,43 | 0,2 | n.b. | 0,9 | 6 | 2 |

Ges.-S = Gesamt-Schwefel, coulometrisch bestimmt nach DIN 51400 Teil 7 Gesamt-Schwefel-Gehalte ≤ 2 ppm wurden bestimmt nach Wickbold (DIN EN 41)
Sulfat-S =Sulfat-Schwefel, ionenchromatographisch bestimmt analog EN ISO 10304-2

**Patentansprüche**

1. Verfahren zur Herstellung eines Ethylamins durch Umsetzung von Ethanol mit Ammoniak, einem primären Amin oder einem sekundären Amin in Gegenwart von Wasserstoff und eines Heterogenkatalysators, **dadurch gekennzeichnet, dass** man ein biochemisch hergestelltes Ethanol (Bio-Ethanol) einsetzt, in welchem zuvor durch In-Kontakt-Bringen mit einem Adsorber Schwefel und/oder schwefelhaltige Verbindungen abgereichert wurden, wobei es sich bei dem Adsorber um ein Kieselgel, ein Aluminiumoxid, einen Zeolith, eine Aktivkohle oder ein Kohlenstoffmolsieb handelt und der Adsorber ein oder mehrere Übergangsmetalle, in elementarer oder kationischer Form, aus den Gruppen VIII und/oder IB des Periodensystems enthält.

2. Verfahren nach dem vorhergehenden Anspruch zur Herstellung von Mono-, Di- und/oder Triethylamin durch Umsetzung des Ethanols mit Ammoniak.

3. Verfahren nach einem der beiden vorhergehenden Ansprüche, **dadurch gekennzeichnet, dass** man ein fermentativ hergestelltes Ethanol einsetzt.

4. Verfahren nach einem der vorhergehenden Ansprüche, **dadurch gekennzeichnet, dass** man Ethanol einsetzt, in welchem zuvor durch In-Kontakt-Bringen mit einem Adsorber $C_{2-10}$-Dialkylsulfide, $C_{2-10}$-Dialkylsulfoxide, 3-Methylthio-1-propanol und/oder S-haltige Aminosäuren abgereichert wurden.

5. Verfahren nach einem der vorhergehenden Ansprüche, **dadurch gekennzeichnet, dass** man Ethanol einsetzt, in welchem zuvor durch In-Kontakt-Bringen mit einem Adsorber Dimethylsulfid abgereichert wurde.

6. Verfahren nach einem der vorhergehenden Ansprüche, **dadurch gekennzeichnet, dass** es sich bei dem Zeolith um einen Zeolith aus der Gruppe natürliche Zeolithe, Faujasit, X-Zeolith, Y-Zeolith, A-Zeolith, L-Zeolith, ZSM 5-Zeolith, ZSM 8-Zeolith, ZSM 11-Zeolith, ZSM 12-Zeolith, Mordenit, beta-Zeolith, Pentasil-Zeolith, Metal Organic Frameworks (MOF) und Mischungen hiervon, die ionen-austauschbare Kationen aufweisen, handelt.

7. Verfahren nach einem der vorhergehenden Ansprüche, **dadurch gekennzeichnet, dass** der Zeolith ein molares $SiO_2/Al_2O_3$-Verhältnis im Bereich von 2 bis 100 aufweist.

8. Verfahren nach einem der vorhergehenden Ansprüche, **dadurch gekennzeichnet, dass** Kationen des Zeoliths ganz oder teilweise gegen Metallkationen ausgetauscht sind.

9. Verfahren nach einem der vorhergehenden Ansprüche, **dadurch gekennzeichnet, dass** der Adsorber Silber und/oder Kupfer enthält.

10. Verfahren nach einem der vorhergehenden Ansprüche, **dadurch gekennzeichnet, dass** der Adsorber 0,1 bis 75 Gew.-% des Metalls bzw. der Metalle enthält.

11. Verfahren nach einem der vorhergehenden Ansprüche, **dadurch gekennzeichnet, dass** das vorherige In-Kontakt-Bringen des Ethanols mit dem Adsorber bei einer Temperatur im Bereich von 10 bis 200°C erfolgte.

12. Verfahren nach einem der vorhergehenden Ansprüche, **dadurch gekennzeichnet, dass** das vorherige In-Kontakt-Bringen des Ethanols mit dem Adsorber bei einem Absolutdruck im Bereich von 1 bis 200 bar erfolgte.

13. Verfahren nach einem der vorhergehenden Ansprüche, **dadurch gekennzeichnet, dass** durch das vorherige In-Kontakt-Bringen des Ethanols mit dem Adsorber Schwefel und/oder schwefelhaltige Verbindungen um ≥ 90 Gew.-% (berechnet S) abgereichert wurden.

14. Verfahren nach einem der Ansprüche 1 bis 12, **dadurch gekennzeichnet, dass** durch das vorherige In-Kontakt-Bringen des Ethanols mit dem Adsorber Schwefel und/oder schwefelhaltige Verbindungen um ≥ 95 Gew.% (berechnet S) abgereichert wurden.

15. Verfahren nach einem der Asprüche 1 bis 12, **dadurch gekennzeichnet, dass** durch das vorherige In-Kontakt-Bringen des Ethanols mit dem Adsorber Schwefel und/oder schwefelhaltige Verbindungen um ≥ 98 Gew.-% (berechnet S) abgereichert wurden.

16. Verfahren nach einem der vorhergehenden Ansprüche, **dadurch gekennzeichnet, dass** durch das vorherige In-Kontakt-Bringen des Ethanols mit dem Adsorber Schwefel und/oder schwefelhaltige Verbindungen auf < 2 Gew.-ppm (berechnet S) abgereichert wurden.

17. Verfahren nach einem der Ansprüche 1 bis 15, **dadurch gekennzeichnet, dass** durch das vorherige In-Kontakt-Bringen des Ethanols mit dem Adsorber Schwefel und/oder schwefelhaltige Verbindungen auf < 1 Gew.-ppm (berechnet S) abgereichert wurden.

18. Verfahren nach einem der Ansprüche 1 bis 15, **dadurch gekennzeichnet, dass** durch das vorherige In-Kontakt-Bringen des Ethanols mit dem Adsorber Schwefel und/oder schwefelhaltige Verbindungen auf < 0,1 Gew.-ppm (berechnet S) abgereichert wurden.

19. Verfahren nach einem der vorhergehenden Ansprüche, **dadurch gekennzeichnet, dass** das vorherige In-Kontakt-Bringen des Ethanols mit dem Adsorber in Abwesenheit von Wasserstoff durchgeführt wurde.

20. Verfahren nach einem der vorhergehenden Ansprüche, **dadurch gekennzeichnet, dass** das eingesetzte Ethanol zuvor in flüssiger Phase mit dem Adsorber in Kontakt gebracht wurde.

21. Verfahren nach einem der vorhergehenden Ansprüche, **dadurch gekennzeichnet, dass** man die Umsetzung des Ethanols mit Ammoniak, einem primären Amin oder einem sekundären Amin bei einer Temperatur im Bereich von 80 bis 300°C durchführt.

22. Verfahren nach einem der vorhergehenden Ansprüche, **dadurch gekennzeichnet, dass** man die Umsetzung des Ethanols mit Ammoniak, einem primären Amin oder einem sekundären Amin in der Flüssigphase bei Drücken im Bereich von 5 bis 30 MPa oder in der Gasphase bei Drücken im Bereich von 0,1 bis 40 MPa durchführt.

23. Verfahren nach einem der vorhergehenden Ansprüche, **dadurch gekennzeichnet, dass** man für die Umsetzung des Ethanols mit Ammoniak, einem primären Amin oder einem sekundären Amin als Heterogenkatalysator einen Hydrier-/Dehydrierkatalysator einsetzt, der ein Metall der Gruppe VIII und/oder IB des Periodensystems enthält.

## Claims

1. A process for preparing an ethylamine by reacting ethanol with ammonia, a primary amine or a secondary amine in the presence of hydrogen and a heterogeneous catalyst, wherein a biochemically prepared ethanol (bioethanol) in which the concentration of sulfur and/or sulfur-comprising compounds has been reduced beforehand by bringing it into contact with an adsorbent is used and the adsorbent is a silica gel, an aluminum oxide, a zeolite, an activated carbon or a carbon molecular sieve and comprises one or more transition metals, in elemental or cationic form, from groups VIII and/or IB of the Periodic Table.

2. The process according to the preceding claim for preparing monoethylamine, diethylamine and/or triethylamine by

reacting ethanol with ammonia.

3. The process according to either of the two preceding claims, wherein an ethanol prepared by fermentation is used.

4. The process according to any of the preceding claims, wherein ethanol in which the concentration of $C_{2-10}$-dialkyl sulfides, $C_{2-10}$-dialkyl sulfoxides, 3-methylthio-1-propanol and/or S-comprising amino acids has been reduced beforehand by bringing it into contact with an adsorbent is used.

5. The process according to any of the preceding claims, wherein ethanol in which the concentration of dimethyl sulfide has been reduced beforehand by bringing it into contact with an adsorbent is used.

6. The process according to any of the preceding claims, wherein the zeolite is a zeolite from the group consisting of natural zeolites, faujasite, X-zeolite, Y-zeolite, A-zeolite, L-zeolite, ZSM 5-zeolite, ZSM 8-zeolite, ZSM 11-zeolite, ZSM 12-zeolite, mordenite, beta-zeolite, pentasil zeolite, metal organic frameworks (MOF) and mixtures thereof which contain ion-exchangeable cations.

7. The process according to any of the preceding claims, wherein the zeolite has a molar $SiO_2/Al_2O_3$ ratio in the range from 2 to 100.

8. The process according to any of the preceding claims, wherein cations of the zeolite have been completely or partly replaced by metal cations.

9. The process according to any of the preceding claims, wherein the adsorbent comprises silver and/or copper.

10. The process according to any of the preceding claims, wherein the adsorbent comprises from 0.1 to 75% by weight of the metal or metals.

11. The process according to any of the preceding claims, wherein the prior contacting of the ethanol with the adsorbent has been carried out at a temperature in the range from 10 to 200°C.

12. The process according to any of the preceding claims, wherein the prior contacting of the ethanol with the adsorbent has been carried out at an absolute pressure in the range from 1 to 200 bar.

13. The process according to any of the preceding claims, wherein the concentration of sulfur and/or sulfur-comprising compound has been reduced by ≥ 90% by weight (calculated as S) by the prior contacting of the ethanol with the adsorbent.

14. The process according to any of claims 1 to 12, wherein the concentration of sulfur and/or sulfur-comprising compound has been reduced by ≥ 95% by weight (calculated as S) by the prior contacting of the ethanol with the adsorbent.

15. The process according to any of claims 1 to 12, wherein the concentration of sulfur and/or sulfur-comprising compound has been reduced by ≥ 98% by weight (calculated as S) by the prior contacting of the ethanol with the adsorbent.

16. The process according to any of the preceding claims, wherein the concentration of sulfur and/or sulfur-comprising compound has been reduced to < 2 ppm by weight (calculated as S) by the prior contacting of the ethanol with the adsorbent.

17. The process according to any of claims 1 to 15, wherein the concentration of sulfur and/or sulfur-comprising compound has been reduced to < 1 ppm by weight (calculated as S) by the prior contacting of the ethanol with the adsorbent.

18. The process according to any of claims 1 to 15, wherein the concentration of sulfur and/or sulfur-comprising compound has been reduced to < 0.1 ppm by weight (calculated as S) by the prior contacting of the ethanol with the adsorbent.

19. The process according to any of the preceding claims, wherein the prior contacting of the ethanol with the adsorbent has been carried out in the absence of hydrogen.

20. The process according to any of the preceding claims, wherein the ethanol used has previously been brought into contact with the adsorbent in the liquid phase.

**21.** The process according to any of the preceding claims, wherein the reaction of the ethanol with ammonia, a primary amine or a secondary amine is carried out at a temperature in the range from 80 to 300°C.

**22.** The process according to any of the preceding claims, wherein the reaction of the ethanol with ammonia, a primary amine or a secondary amine is carried out in the liquid phase at pressures in the range from 5 to 30 MPa or in the gas phase at pressures in the range from 0.1 to 40 MPa.

**23.** The process according to any of the preceding claims, wherein the heterogeneous catalyst used for the reaction of the ethanol with ammonia, a primary amine or a secondary amine is a hydrogenation/dehydrogenation catalyst comprising a metal of group VIII and/or IB of the Periodic Table.

**Revendications**

**1.** Procédé pour la préparation d'une éthylamine par transformation d'éthanol avec de l'ammoniac, une amine primaire ou une amine secondaire en présence d'hydrogène et d'un catalyseur hétérogène, **caractérisé en ce qu'**on utilise un éthanol préparé par voie biochimique (bioéthanol) dans lequel on a appauvri au préalable le soufre et/ou les composés soufrés par mise en contact avec un adsorbant, où il s'agit, pour l'adsorbant, d'un gel silicique, d'un oxyde d'aluminium, d'une zéolithe, d'un charbon actif ou d'un tamis moléculaire carboné et l'adsorbant contient un ou plusieurs métaux de transition, sous forme élémentaire ou cationique, des groupes VIII et/ou IB du système périodique.

**2.** Procédé selon la revendication précédente pour la préparation de monoéthylamine, de diéthylamine et/ou de triéthylamine par transformation de l'éthanol avec de l'ammoniac.

**3.** Procédé selon l'une quelconque des deux revendications précédentes, **caractérisé en ce qu'**on utilise un éthanol préparé par fermentation.

**4.** Procédé selon l'une quelconque des revendications précédentes, **caractérisé en ce qu'**on utilise de l'éthanol, dans lequel on a appauvri au préalable, par mise en contact avec un adsorbant, les sulfures de $C_{2-10}$-dialkyle, les sulfoxydes de $C_{2-10}$-dialkyle, le 3-méthylthio-1-propanol et/ou les aminoacides contenant du soufre.

**5.** Procédé selon l'une quelconque des revendications précédentes, **caractérisé en ce qu'**on utilise de l'éthanol, dans lequel on a appauvri au préalable, par mise en contact avec un adsorbant, le sulfure de diméthyle.

**6.** Procédé selon l'une quelconque des revendications précédentes, **caractérisé en ce qu'**il s'agit, pour la zéolithe, d'une zéolithe du groupe formé par les zéolithes naturelles, la faujasite, la zéolithe X, la zéolithe Y, la zéolithe A, la zéolithe L, la zéolithe ZSM 5, la zéolithe ZSM 8, la zéolithe ZSM 11, la zéolithe ZSM 12, la mordénite, la zéolithe bêta, la zéolithe pentasile, les cadres organiques métalliques (Metal Organic Frameworks - MOF) et leurs mélanges, qui présentent des cations échangeables contre des ions.

**7.** Procédé selon l'une quelconque des revendications précédentes, **caractérisé en ce que** la zéolithe présente un rapport molaire $SiO_2/Al_2O_3$ dans la plage de 2 à 100.

**8.** Procédé selon l'une quelconque des revendications précédentes, **caractérisé en ce que** les cations de la zéolithe sont échangés totalement ou partiellement contre des cations métalliques.

**9.** Procédé selon l'une quelconque des revendications précédentes, **caractérisé en ce que** l'adsorbant contient de l'argent et/ou du cuivre.

**10.** Procédé selon l'une quelconque des revendications précédentes, **caractérisé en ce que** l'adsorbant contient 0,1 à 75% en poids du métal ou des métaux.

**11.** Procédé selon l'une quelconque des revendications précédentes, **caractérisé en ce que** la mise en contact préalable de l'éthanol avec l'adsorbant a été réalisée à une température dans la plage de 10 à 200°C.

**12.** Procédé selon l'une quelconque des revendications précédentes, **caractérisé en ce que** la mise en contact préalable de l'éthanol avec l'adsorbant a été réalisée à une pression absolue dans la plage de 1 à 200 bars.

**13.** Procédé selon l'une quelconque des revendications précédentes, **caractérisé en ce que** par la mise en contact préalable de l'éthanol avec l'adsorbant, le soufre et/ou les composés soufrés ont été appauvris à raison de ≥ 90% en poids (calculé sous forme de S).

**14.** Procédé selon l'une quelconque des revendications 1 à 12, **caractérisé en ce que** par la mise en contact préalable de l'éthanol avec l'adsorbant, le soufre et/ou les composés soufrés ont été appauvris à raison de ≥ 95% en poids (calculé sous forme de S).

**15.** Procédé selon l'une quelconque des revendications 1 à 12, **caractérisé en ce que** par la mise en contact préalable de l'éthanol avec l'adsorbant, le soufre et/ou les composés soufrés ont été appauvris à raison de ≥ 98% en poids (calculé sous forme de S).

**16.** Procédé selon l'une quelconque des revendications précédentes, **caractérisé en ce que** par la mise en contact préalable de l'éthanol avec l'adsorbant, le soufre et/ou les composés soufrés ont été appauvris à < 2 ppm en poids (calculé sous forme de S).

**17.** Procédé selon l'une quelconque des revendications 1 à 15, **caractérisé en ce que** par la mise en contact préalable de l'éthanol avec l'adsorbant, le soufre et/ou les composés soufrés ont été appauvris à < 1 ppm en poids (calculé sous forme de S).

**18.** Procédé selon l'une quelconque des revendications 1 à 15, **caractérisé en ce que** par la mise en contact préalable de l'éthanol avec l'adsorbant, le soufre et/ou les composés soufrés ont été appauvris à < 0,1 ppm en poids (calculé sous forme de S).

**19.** Procédé selon l'une quelconque des revendications précédentes, **caractérisé en ce que** la mise en contact préalable de l'éthanol avec l'adsorbant a été réalisée en l'absence d'hydrogène.

**20.** Procédé selon l'une quelconque des revendications précédentes, **caractérisé en ce que** l'éthanol utilisé a été mis en contact en phase liquide au préalable avec l'adsorbant.

**21.** Procédé selon l'une quelconque des revendications précédentes, **caractérisé en ce qu'**on réalise la transformation de l'éthanol avec de l'ammoniac, une amine primaire ou une amine secondaire à une température dans la plage de 80 à 300°C.

**22.** Procédé selon l'une quelconque des revendications précédentes, **caractérisé en ce qu'**on réalise la transformation de l'éthanol avec de l'ammoniac, une amine primaire ou une amine secondaire en phase liquide à des pressions dans la plage de 5 à 30 MPa ou en phase gazeuse à des pressions dans la plage de 0,1 à 40 MPa.

**23.** Procédé selon l'une quelconque des revendications précédentes, **caractérisé en ce qu'**on utilise pour la transformation de l'éthanol avec de l'ammoniac, une amine primaire ou une amine secondaire comme catalyseur hétérogène un catalyseur d'hydrogénation/déshydrogénation qui contient un métal du groupe VIII et/ou IB du système périodique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO A A
- WO 2003020850 A
- US 2003070966 A1
- US 2003113598 A1
- US 6531052 B1
- EP 0101254 A
- WO 03020850 A2
- DE 1953263 A
- EP 382049 A
- EP 963975 A
- EP 696572 A
- EP 1270543 A1
- DE 10261195
- US 3275554 A
- DE 2125039 A
- DE 3611230 A


### In der Beschreibung aufgeführte Nicht-Patentliteratur


- Ullmann's Encyclopedia of Industrial Chemistry. 2000
- *CHEMICAL ABSTRACTS,* 102: 222463
- **M.Kh. Annagiev et al.** *Doklady - Akademiya Nauk Azerbaidzhanskoi SSR,* 1984, vol. 40 (12), 53-6
- Ullmann's Encyclopedia. 2000
- Kirk-Othmer Encyclopedia of Chemical Engineering. Wiley, 1995, vol. 16
- Catalysis and Zeolites. Springer, 1999
- **Li et al.** *Nature,* 1999, vol. 402, 276-279
- **Larsen et al.** *J. Chem. Phys.,* 1994, vol. 98, 11533-11540
- *J. Mol. Catalysis A,* 2003, vol. 21, 237-246
- **A.J. Hernandez-Maldonado et al.** *Ind. Eng. Chem. Res.,* 2003, vol. 42, 123-29
- **T.R. Felthouse et al.** *J. of Catalysis,* 1986, vol. 98, 411-33
- **K. Weissermel ; H.-J. Arpe.** Industrial Organic Chemistry. Wiley-VCH, 2003, 194
- Electronic Version of Sixth Edition of Ullmann's Encyclopedia of Industrial Chemistry. 2000